# EUROPEAN PATENT SPECIFICATION

(11) **EP 1 608 360 B1**
(45) Date of publication and mention of the grant of the patent: **03.01.2007**
(21) Application number: 03717533.8
(22) Date of filing: 31.03.2003
(51) Int. Cl.: A61K 31/341, A61K 31/19, A61P 5/48

(54) **SYNERGISTIC HEPATOPROTECTIVE COMPOSITION AND A METHOD THEREOF**
SYNERGISTISCHE LEBERSCHÜTZENDE ZUSAMMENSETZUNG UND VERFAHREN DAZU
COMPOSITION HEPATOPROTECTRICE SYNERGIQUE ET METHODE CORRESPONDANTE

(43) Date of publication of application: 28.12.2005
(73) Proprietor: COUNCIL OF SCIENTIFIC AND INDUSTRIAL RESEARCH, New Delhi 100 001 (IN)
(72) Inventor: GUPTA, Vishwa Nath, Jammu 180 001 (IN); SURI, Krishan, Avtar, Jammu 180 001 (IN); GUPTA, Bishan, Datt, Jammu 180 001 (IN); JAGGI, Bupinder Singh, Jammu 180 001 (IN); SATTI, Naresh, Kumar, Jammu 180 001 (IN); CHANDAN, Bal, Krishan, Jammu 180 001 (IN); SHARMA, Neelam, Jammu 180 001 (IN); BHARDWAJ, Vikram, Jammu 180 001 (IN); SURI, Om, Parkash, Jammu 180 001 (IN); QAZI, Ghulam, Nabi, Jammu 180 001 (IN); SURI, Jyotsna, Jammu 180 001 (IN); BARGOTRA, Madalsa, Jammu 180 001 (IN)
(74) Representative: Schwarz, Albin
(86) International application number: PCT/IN2003/000129
(87) International publication number: WO 2004/087127

(56) References cited:
- WO-A-01/85710
- US-A1- 2002 068 098

## Description

### Field of the present invention

The present invention relates to a synergistic formulation comprising trans-tetracos-15-enoic acid (t-TCA) and andrographolide (AP) in the ratio ranging between 1:7 to 7:1 (w/w), useful in hepatoprotection; and a process for the preparation of a synergistic formulation comprising trans-tetracos-15-enoic acid (t-TCA) and andrographolide (AP) useful in hepatoprotection, said process comprising steps of grounding the particles of the t-TCA and AP into fine particles, mixing the fine particles in ratio ranging between 1:7 to 7:1 (w/w), and grinding the mixture to obtain formulation.

### Background and prior art references of the present invention

Literature survey revealed that earlier reports showed the presence of trans-tetracos-15-enoic acid in Jojoba oil ex. *Simmondsia chinesis* seeds (0.62-1.11%), cis isomer of the acid is reported in the fatty acids of the seeds oil of *Microula sikkimensis* (1.2 %) [Wang, Huiying ; Yu, Xuejian; yi, Yuanfen and Ding, Jingkai, Yunnan Zhiwu Yanjiu 1989, **11** (I), 60-4 (Ch)., Li, Jingmin; Wang Jingpin, Yu, Penglan. Zhiwu Xuebao, 1989, **31** (1), 50-3 (Ch.)]. These reports do not mention isolation of the constituents and the content estimation is based on GLC data. *Indigofera tinctoria* has been in use in indigenous system of medicine in epilepsy, nervous disorders and bronchitis [Wealth of India, vol. 5, (Council of Scientific and Industrial Research, New Delhi) 182, (1959)]. The plant is also used as ointments in sores, old ulcers and haemorrhoids [R.N. Chopra, S.L, Nayar and I.C. Chopra, Glossary of Indian Medicinal plants, 141 (1956)].

The leaves of the plants have been used in liver ailments [Nadkarni, K.M., Indian Materia Medica, Vol.1 (Popular Book Depot, Bombay), 680 (1954)]. Extract of the leaves of the plant has exhibited marked hepatoprotective effect against CCl₄ induced hepatic injury in rabbits, rats and mice at RRL-Jammu [K.K. Anand, Dewan Chand, B.J. Ray, Ghatak, Indian J. Expl. Biol., **17**, 685, (1979); K.K. Anand, Dewan Chand, B.J. Ray, Ghatalc and RK. Arya, Indian J. Expl. Biol., **19**, 298 (1981)]. Recent study in RRL Jammu for hepatoprotective effect of the plant extract and further bioactivity guided fractionation has been reported, which has further resulted in identification of *trans*-tetracos-15-enoic acid as the active principle. [379/DEL/2000, B.Singh, A.K. Saxena, B.K. Chandan, V. Bhardwaj, V.N. Gupta and O.P. Suri, Phytother. Res., **15**, 294-297, (2001)]. *trans*-tetracos-15-enoic acid has been synthesised and observed to possess dose related hepatoprotective effect against, Galactosamine, paracetamol and CCl₄ as hepatotoxins using commercially available silymarin as reference material [36/DEL/2000].

Andrographolide, a major bicyclic diterpenoid lactone has shown to possess multiple pharmacological activities such as reduction in hexabarbital or phenobarbital sleeping time, [B R Chaudhary, S J Hague, and M K Poddar; *Planta Medica* , **53**, 135-140 (1987); B Roy, M K.Poddar, B.Shukla, P K S Visen, G K Patnaik, and B N Dhawan., *Planta Medica*, **58**, 146-149 (1992)]. Andrographolide, the active constituent isolated from the plant *Andrographis paniculata,* showed a significant dose depended protective activity against paracetamol induced toxicity on *ex vivo* preparation of isolated rat hepatocytes.

Andrographolide was found to be more potent than silymarin, a standard hepatoprotective agent [P.K. Visen, B Shukla, G K Patnaik, B.N. Dhawan *J*. *Ethanopharmacol*, **40** (2); 131-136 (1993)]. Incidence of colds in andrographis treated subject was 30% as compared to 62% in the control group. D.D. Cacers, J.L. Hancke, R.A. Burgos and G.K. Wikman, *Phytomedicine* **4** (2), 101-104 (1997)]. Controlled studies involving over 500 subjects indicate that *Andrographis paniculata* is effective at reducing the prevalence and intensity of colds and sinusitis and shortening the duration of symptoms (J Melchior, S Palm and G. Wikman ; *Phytomedicine*, **3** (4), 315-318, (1996) ; D.D. Caceres, J.L. Hancke, R.A. Burgos, *et al*., *Phytomedicine* **6**(4),217-223, (1999)].

Andrographis is an immune booster, a possibility supported by the fact that it stimulates several immune parameters in mice. The results suggest that extracts are more potent than purified andrographolide [A Puri, R Saxena, R.P. *Saxena, et al, J. Nat. Prod.* **56**, 995-999 (1993)]. Research suggested that andrographolides have a direct antiviral effect and a direct antiparasitic effect but not a antibacterial effect. [R S Chang, L Ding, G . *Chem et al.,* Proc. Soc. Expl. Biol. Med. **197**, 59-66 (1991), R.K. Raj, *Indian J. Physiol Pharmacol,* **19**, (1), (1975), A Leelarasamee, S. Trakulsomboon, N. Sittisomwong *J*. *Med. Assoc. Thal*, **73**, 299-304 (1990)]. In an animal model, andrographolide was shown to be twice as effective as fish oil in preventing the incidence and severity of restinosis following Angio plasty. D.W. Wang, H.Y. Jhao, J. Tongji Med. Univ. **13** (4), 193-198 (1993)].

The mechanisms may be the antithrombotic effect of Andrographis, which may occur as a result of decreases in thromboxane and platelet aggregation [Hy Zhao, and Wy. Feng *Chin Med. J* (Engl.) **104** (9), 770-771, (1991)]. *Andrographis paniculata* has also been shown to be a liver protecting substance. Andrographis is hepatoprotective in mice treated with CCl₄ or tert butyl hydroperoxide, both highly toxic compounds. Andrographis was also found to the superior to silymarin in protecting the liver against paracetamol toxicity and against paracetamol and galactosamine. [A. Kapil, I. B. Koul, S.K., Banerjee B.D. Gupta, *Biochem Pharmacol* , **46** (I) 182-185, 1993 ; P.K. Visen, B. Shukla, G.K. Patnaik, B.N. Dhawan *J*. *Ethanopharmacol,* **40** (2) 131-136, (1993)]. S.S. Handa and A. Sharma , *Indian J*. *Med*. *Res*. **92**, 284-292, (1990)].

Andrographolides are highly bioavailable in humans [A. Pannossian, *et al*. *Phytomedicine*, **7** (5), 351-364, (2000)]. Labelled andrographolide is readily distributed through out the body. After 72 hrs, nearly 90% of andrographolides are excreted mostly by urinary excretion [Wuxi Medicine Institute, Sushow Medical Academy, Acta Biochemica, Biophysica sinica , **11**,1979 (no pages listed). There have been reports in the chinese medical literature of administration of high doses of *Andrographis paniculata* in animals and humans.

[F. Sandberg Andrographis herba Chuanxinlian A Review, American Botanical Council, Austin, Texas, USA (1994)]. Although no systematic long term studies have been done in humans. Subjects given andrographis at recommended doses had no change in hepatic or a renal function, blood cell count or blood chemistries [J. Hancke, R. Burgos, D.Caceres, G. Wilkman, *Phytotherapy Research,* **9**, 559-562 (1995)]. According to Barilla, humans have also been treated with high levels [J. Barilla , *Andrographis paniculata.* Keats publishing, Los Angelos, CA USA (1999)]. Andrographis has clear antifertility effects in experimental animals both in males and in females. Male rats become infertile at intake of 20 mg/day and female rats become infertile at higher doses. [M. Akbarsha, B. Manivannan, H.K. Shahul, *et al*, *Indian J. Exp. Biol.* **28**, 421-426 (1990); M.S. Zoha, A.H.B. Hussain, S.A.R. Choudhary, Bangladesh, *Med. Res. Council Bull,* **15**, 34-37 (1989); F.Sandberg, Andrographis herba Chu anxinlian, A Review, American Botanical Council, Austin , Texas, USA (1994)].

### Objects of the present Invention

The main object of the present invention is to develop a synergistic formulation for the hepatoprotection.

Another object of the present invention is to develop a process for the preparation of a synergistic formulation for hepatoprotection.

Still another object of the present invention is to develop a safe and efficient hepatoprotection formulation.

### Summary of the present invention

The present invention relates to a synergistic formulation comprising trans-tetracos-15-enoic acid (t-TCA) and andrographolide (AP) in the ratio ranging between 1:7 to 7:1 (w/w), useful in hepatoprotection; and a process for the preparation of a synergistic formulation comprising trans-tetracos-15-enoic acid (t-TCA) and andrographolide (AP) useful in hepatoprotection, said process comprising steps of grounding the particles of the t-TCA and AP into fine particles, mixing the fine particles in ratio ranging between 1:7 to 7:1 (w/w), and grinding the mixture to obtain formulation.

### Detailed description of the present invention

Accordingly, the present invention relates to a synergistic formulation comprising trans-tetracos-15-enoic acid (t-TCA) and andrographolide (AP) in the ratio ranging between 1:7 to 7:1 (w/w), useful in hepatoprotection; and a process for the preparation of a synergistic formulation comprising trans-tetracos-15-enoic acid (t-TCA) and andrographolide (AP) useful in hepatoprotection, said process comprising steps of grounding the particles of the t-TCA and AP into fine particles, mixing the fine particles in ratio ranging between 1:7 to 7:1 (w/w), and grinding the mixture to obtain formulation.

In an embodiment of the present invention, wherein a synergistic formulation comprising trans-tetracos-15-enoic acid (t-TCA) and andrographolide (AP) in the ratio ranging between 1:7 to 7:1 (w/w), useful in hepatoprotection

In another embodiment of the present invention, wherein the ratio of t-TCA and AP is 1:1 (w/w).

In yet another embodiment of the present invention, wherein the constituents of the formulation can be either herbal or synthetic.

In still another embodiment of the present invention, wherein the formulation shows therapeutic index of more than 40.

In still another embodiment of the present invention, wherein a process for the preparation of a synergistic formulation comprising trans-tetracos-15-enoic acid (t-TCA) and andrographolide (AP) useful in hepatoprotection, said process comprising steps of:
- grounding the particles of the t-TCA and AP into fine particles,
- mixing the fine particles in ratio ranging between 1:7 to 7:1 (w/w), and
- grinding the mixture to obtain formulation.

In still another embodiment of the present invention, wherein grounding the particles for about 20 minutes at about 100-120 rotation/minute.

In still another embodiment of the present invention, wherein grinding the mixture for about 15 minutes at about 70-80 rotations/minute.

In still another embodiment of the present invention, wherein the formulation shows the mesh size of about less than 100-mesh.

In still another embodiment of the present invention, wherein the constituents of the formulation can be either herbal or synthetic.

To develop a hepatoprotective Simulation based on chemical constituents of herbal origin. One of which i.e., *trans*-tetracos-15-enoic acid has shown significant activity in restoration of altered levels of transaminases, bilirubin, triglycerides, ALP and GSH due to hepatotoxicants and other one possesses multiple pharmacological activities such as reduction in hexabarbital or Phenobarbital sleeping time, antiperoxidative potential in the liver. These investigations have substantiated the therapeutic potential of andrographolide. In the mixture, both the aspects of hepatoprotective activity get boosted which can be apparently varified by a glance through tables (1-4) indicating reversal of effect of one hepatotoxin i.e., paracetamol employed in the evaluation. To provide a chemically defined drug for use in various types of liver disorders.

The present invention relates to a process for development of the synergistic hepatoprotective composition containing *trans-*tetracos-15-enoic acid (TCA) and andrographolide (AP). The invention particularly relates to the mixing of *trans*-tetracos-15-enoic acid and andrographolide of synthetic or of plant origin ranging from 7:1 of *trans*-tetracos-15-enoic acid to 1: 7 of andrographolide.

Accordingly, the present invention' provides a process for synergistic hepatoprotective pharmaceutical composition which comprises mixing of t-TCA and Andrographolide in the ratio of 7 : 1 to 1 :7. The formulation has been so developed that it shall be of potential therapeutic application in liver ailments.

In the embodiment of the present invention we are describing a process for a synergistic hepatoprotective formulation in which t-TCA and Andrographolide are mixed in the range from 7 :1 and 1:7 respectively.

In another embodiment of the invention a formulation has been prepared which has exhibited enhanced hepatoprotective activity than the individual constituents viz., t-TCA and andrographolide.

In yet another embodiment of the invention, the formulation described herein shows more and broader spectrum hepatoprotective activity than the established hepatoprotective agents such as silymarin.

Pharmacological evaulation shows the formulation to possess synergistic action in a broad spectrum of hepatoprotective activity parameters as monitored in a series of biochemical estimations against paracetamol as hepatotoxin using silymarin as reference material.

A comparison with known hepatoprotective agent silymarin, native t-tetracos-15-enoic acid and andrographolide revealed that formulation HV-392 exhibited higher hepatoprotective potential than the individuals, which can be easily verified by a glance through (tables 3 to 4) ith respect to the effect on the formulation of the degradation products of lipid Peroxidation, release of glutamic transaminases (GPT and GOT), alkaline Phosphatase (ALP) bilirubin, triglycesides (TG) and reduced glutathione (GSH) status.

Treatment of experimental animals with the formulation using paracetamol as hepatoxin reduced the elevated levels of serum GPT, GOT, ALP, Bilirubin, T.G. and increased GSH levels.

It may be mentioned that the formulation showed better reversal of action of hepatotoxins in 50 mg/kg-1 dose having 25 mg of each constituent than that observed with 50 mg kg-1 dose of each of constituents.

All the list materials were administered P.O. routes in a uniform dose level of 50 mg kg-1. Formulation is extremely safe having a therapeutic index of > 40. No mortality was recorded upto a dose of 2g-kg-1 p.o. in mice.

The invention is described with reference to the examples given below which should not, however be construed to limit the scope of present invention.

### Example 1

### Preparation of the formulation

The compounds viz., *trans*-tetracos-15-enoic acid (TCA) and andrographolide were ground finely in a pestle mortar for 20 minutes at 100-120 rotation-minute⁻¹, mixed in the desired ratio i.e., 7:1 to 1:7 (w/w) coded as HV-407 to HV-412 respectively and 1:1 (w/w) as HV-392 and subjected to grinding for 15 minutes at 70-80 rotations per minute. The formulation was passed through a 100 -mesh sieve repeatedly for uniformity.

### Example 2

### Pharmacological evaluation data of the formulation HV-407 (Table 1).

Animals on treatment with the formulation using Paracetamol (APAP) as hepatotoxin reduced the elevated levels of serum GPT , GOT, ALP, Albumin, T G and Lipid peroxidation 51.86, 54.04, 56.75, 44.77, 55.24% and 45.99% respectively and increased GSH level to 60.60. The corresponding parameters with silymarin being 58.09, 54.06, 54.20, 50.26, 50.60, 55.78 and 51.98 with andrographolide 54.15, 60.14, 54.97, 63.88, 66.41, 58.70, 58.35 and 57.35 with *trans*-tetracos-15-enoic acid 75.00, 62.00, 82.35, 56.35, 66.45, 64.15 and 53.15% respectively. The said formulation in the above mentioned ratio has reduced the tested serum level much less as compared to the individual compounds.

### Example 3:

### The formulation HV-408 (Table 1)

The change in the serum levels with paracetamol as toxin recorded on the administration of the formulation are:
GPT (49.46%), GOT (44.14%), ALP (48.57%), Albumin (51.73%), T G (40.14%), LP) (51.23%) and GSH (50-02%), respectively. The corresponding parameter with silymarin ⁽¹⁾, andrographolide ⁽²⁾ and t-TCA ⁽³⁾ being (Table 1, 2)
   (1) 58.9, 54.06, 54.20, 50.26 , 50.26, 50.60, 57.58 and 51.98
   (2) 54.15, 60.14, 66.41, 58.70, 55.35 and 57.35
   (3) 75.00, 62.00, 82.35, 56.35, 66.45, 64.15 and 53.15

### Example 4

### Formulation HV-409

Treating experimental animals with the formulation with paracetamol as liver toxin reduced elevated levels of serum GPT (34.61%), GOT (39.76%), ALP (38.64%), Albumin (28.02%), TG (27.85%), LP (31.31%) where as reduced level of GSH was increased by 52.82% respectively , whereas corresponding parameters with silymarin, *trans*-tetracos-15-enoic acid and andrographolide were observed as given in table (1 & 2).

### Example 5

### Formulation HV-392 (Table 2, 3 and 4)

The change in serum levels with paracetamol as hepatic toxicant recorded on the administration of the formulation are:
GPT (80.92%), GOT (70.99%), IDH (81.01%), ALP (84.77%)
TG (59.74%), Albumin (88.45%), LP (76.98%), GSH (73.99%) and protein (56.03%) respectively. Corresponding parameters with silymarin⁽¹⁾, Andrographolide⁽²⁾ and t-tca⁽³⁾ being
   1). 59.64, 56.64, 57.69, 51.49, 54.46, 48.46, 56.42, 54.82 and 46.79
   2). 54.15, 60.14, 54.97, 63.88, 58.70, 66.41, 58.35, 57.35, 52.27
   3). 57.99, 54.92, 52.96, 56.35, 66.05, 55.48 and 42.35 respectively.

However it may be emphasized that 1:1 formulation (w/w) showed the best reversal of action of hepatotoxin (Paracetamol) at dose level of 50 mg Kg⁻¹ as compared to 50 mg Kg⁻¹ dose of individual constituents.

### Advantages of the present invention

Most of the hepatoprotective herbal preparations /formulation available in the market are not standardized chemically as well as biologically. Efficacy of the herbal formulation are known to be dependent upon secondary, metabolites and reliability of the formulation can only be assured if batch-to-batch standardization (chemical and pharmacological are carried out).

In the present invention
(a) Chemical composition of the formulation is well described, hence reproducible biological activity is assured.
(b) Pharmacological evaluation data of the formulation clearly indicates synergistic action of the constituents of the formulation.

### Physiological effects of the synergistic hepatoprotective formulation

In the curative group, moderate to marked liver damage was seen in all the cases (As APAP was given first) but no evidence of regeneration was seen with lower dose of 1:1 hepatoprotective mixture at 6.25 mg/kg. Marginal regeneration was observed with dose of 25 mg/kg. Regeneration was also seen with a dose of 50 mg/kg of 1:1 hepatoprotective mixture of TCA + Andrographolide. This was assessed as mild increased basophillia of regenerating hepatocytes, binucleation , multinucleation and mitosis. This area of regeneration remained localised at the site of centrilobular necrosis.

### Histopathological parameters

1. Piecemeal necrosis of hepatocytes in acinar zone 3 i.e., around central veins (also k/a centrilobular necrosis). This is characteristic liver injury caused by paracetamol administration.
2. Bridging necrosis i.e., wide bands of necrosed hepatocytes forming bridges between the two adjoining central veins or adjacent central vein and portal area.
3. Spotty Necrosis.
4. Ballooning degeneration of hepatocytes.
5. Lobular Disarray.
6. Chronic inflammatory cell infiltrate.

Liver damage caused by paracetamol has been assessed in decreasing severity as Marked, Moderate, Mild, Absent.

In prophylactic study group the reversal of above parameters was investigated after treatment with curative doses hepatoprotective mixture of TCA and Andrographolide (1:1 w/w).

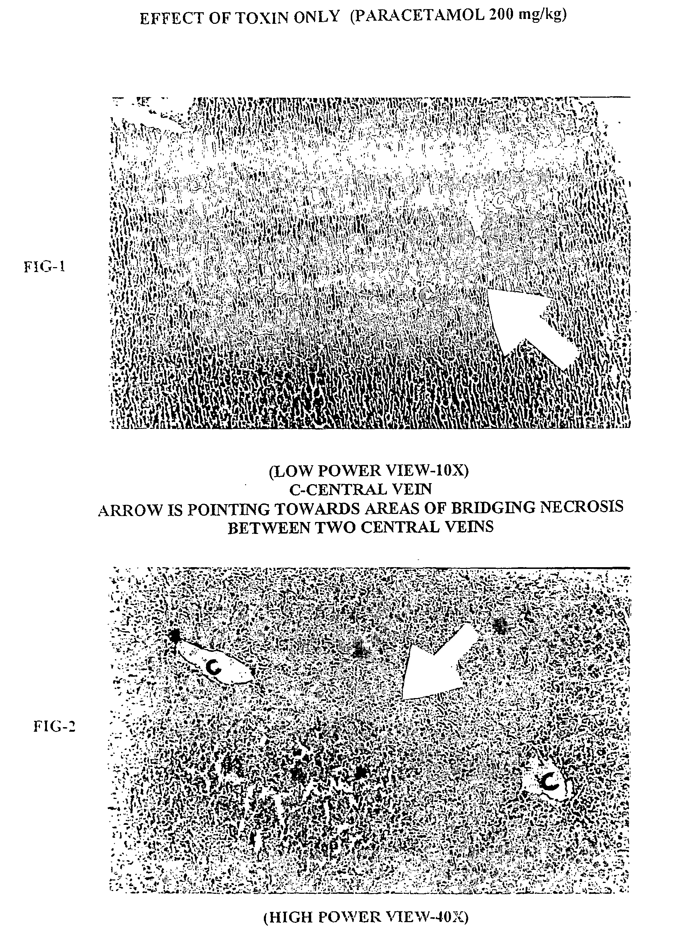

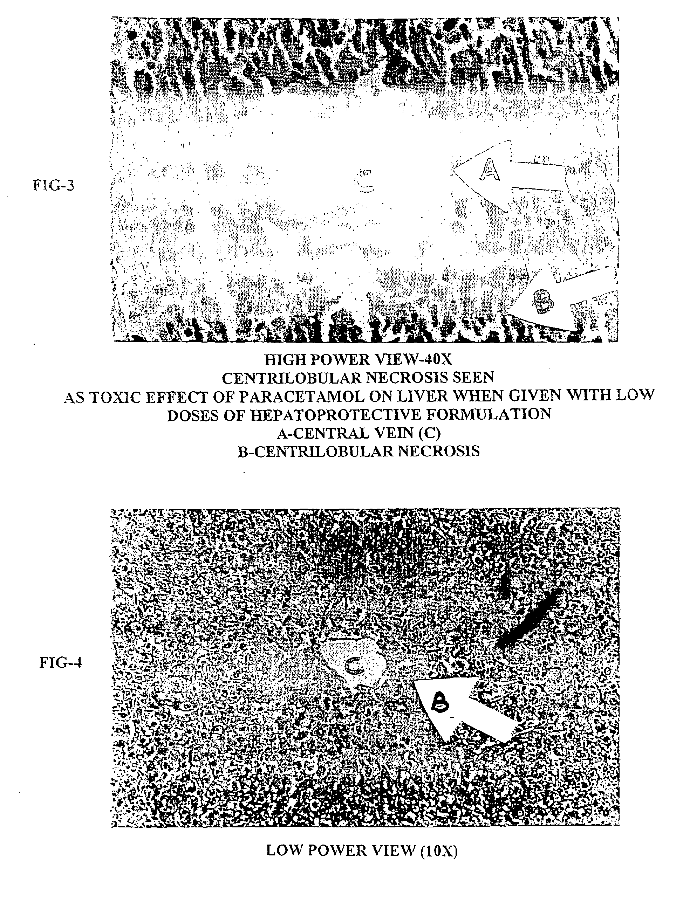

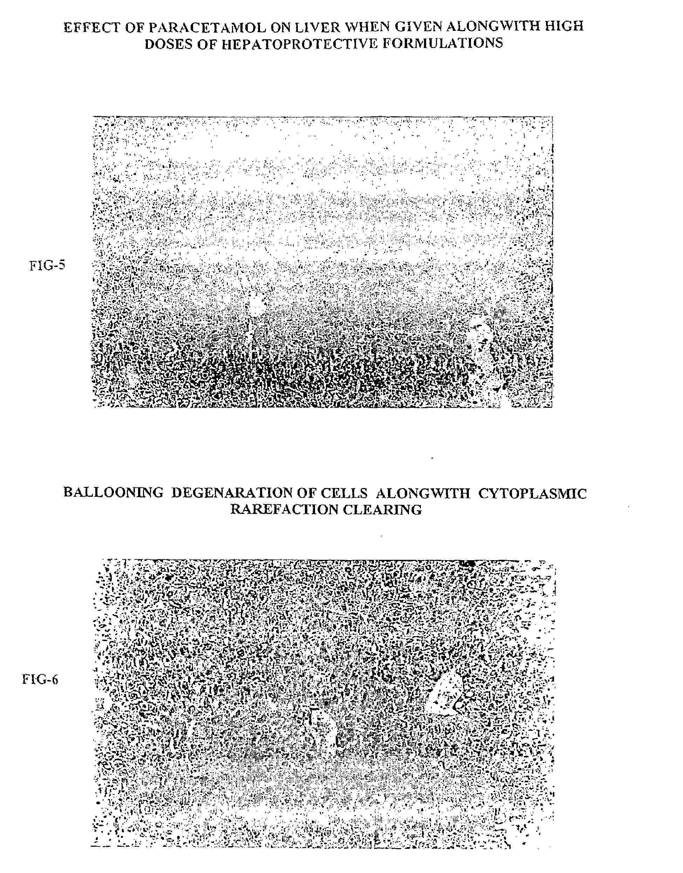

In curative study group, the regenerative effect of the hepatoprotective mixture was assessed under similar conditions (Table 5 and figures 1 to 6)

### Brief description of the accompanying drawings:

**Fig.1** Low Power view 10 X of bridging necroses
**Fig.2** High Power view 40X of bridging necroses
**Fig.3** High Power view 40X of centrilobular necrosis
**Fig.4** Lower Power view 10X of centrilobular necrosis
**Fig.5** Effect of paracetamol on liver when given along with high doses of hepatoprotective formulations
Fig.6 **Ballooning degeneration of cells along with cytoplasmic rarefaction clearing**

**Table 1: Hepatoprotective activity (in vivo) of synergistic formulation and Silymarin agains paracetamol (APAP) induced hepatic injury in mice². (prophylactic study)**

| **Treatment** | Dose | Serum parameters | | | | | Hepatic parameters | |
|---|---|---|---|---|---|---|---|---|
| | mg/kg p.o. | GPT (Units) | GOT (Units) | ALP^{b} | S. Albumin g % | Triglycerides (mg %) | Lipid peroxidation^{c} | Glutathione^{d} |
| Vehicle only | - | 133.00±6.13 | 141.74±4.95 | 46.87±2.41 | 4.02±0.09 | 39.26±2.30 | 40.98±1.55 | 9.03±0.46 |
| Vehicle + APAP | - | 1860.74±61.98 | 1376.47±42.59 | 84.53±4.10 | 2.20±0.13 | 73.40±3.28 | 76.47±3.61 | 3.00±0.12 |
| HV 407 + APAP | 50 | 964.65±41.11 | 709.08±33.09 | 63.524±2.09 | 3.01±0.18 | 54.54±2.18 | 60.18±2.65 | 6.65±0.20 |
| | | (51.86±2.38) | (54.04±2.67) | (55.77±5.55) | (44.77±9.88) | (55.24±6.38) | (45.90±7.45) | (60.60±3.37) |
| HV 408+APAP | 50 | 1006.14±53.42 | 831.29±46.19 | 66.24±3.32 | 3.14±0.12 | 59.69±2.19 | 58.28±2.38 | 6.02±0.31 |
| | | (49.46±3.09) | (44.14±3.74) | (48.57±8.80) | (51.73±7.10) | (40.14±6.44) | (51.23±6.71) | (50.02±5.18) |
| HV 409 + APAP | 50 | 1262.50±52.38 | 891.40±32.52 | 70.14±4.62 | 2.71±0.15 | 63.89±2.33 | 65.35±1.99 | 6.06±0.29 |
| | | (34.61±3.03) | (39.76±2.66) | (38.64±12.28) | (28.02±8.36) | (27.85±6.83) | (31.31±5.60) | (50.82±4.81) |
| HV 410 + APAP | 50 | 755.00±34.26 | 617.45±50.25 | 56.50±2.60 | 3.25±0.20 | 52.81±2.08 | 51.69±2.61 | 7.42±0.27 |
| | | 63.99±1.98) | (61.46±4.06) | (74.43±6.91) | (57.50±11.09) | (60.30±6.08) | (69.80±7.34) | (73.21±4.57) |
| HV 411 + APAP | 50 | 882.75±52.04 | 765.29±36.65 | 64.45±3.63 | 2.89±0.07 | 59.54±1.26 | 55.66±3.27 | 6.66±0.17 |
| | | 56.61±3.01) | (49.49±2.96) | (53.30±9.64) | (37.89±4.27) | (40.58±3.68) | (53.63±9.22) | (60.66±2.90) |
| HV 412 + APAP | 50 | 921.91±42.84 | 817.79±41.73 | 65.31±3.22 | 2.93±0.11 | 63.73±2.63 | 55.66±2.60 | 6.15±0.25 |
| | | 54.33±2.47) | (45.24±3.37) | (51.03±8.53) | (40.47±6.32) | (28.31±7.72 | (58.62±7.34) | (52.29±4.27) |
| Silymarin + | 50 | 856.95±23.76 | 708.85±41.24 | 64.11±3.26 | 3.11±0.11 | 56.12±2.43 | 56.03±2.38 | 6.14±0.26 |
| APAP | | 58.09±1.37) | (54.06±3.34) | (54.20±8.66) | (50.26±6.43) | (50.60±7.12) | (57.58±6.72) | (51.93±4.44) |

| | | | | | | | | |
|---|---|---|---|---|---|---|---|---|
| a: Values represent the mean ± SE and within parentheses percent hepatoprotective activity of six animals in each group. H: Hepatoprotective activity was calculated as {1- (T - V / C - V)} x 100 where "T" is mean value of drug and APAP, " C" is mean value of APAP alone and "V" is the mean value of vehicle treated animals. Unit: each unit is µmole pyruvate/min/L. b: is µ mole of *p*-nitrophenol formed/min/ L, c: is n moles MDA/g liver., d: is µ mole GSH/g liver | | | | | | | | |

**Table-2: Hepatoprotective potential of Andrographolide (AP) against Paracetamol (APAP) induced hepatic injury in Rodents (prophylactic).**

| Treatment | Dose mg kg⁻¹ | Serum parameter^{a} | | | | | | Hepatic parameters | | |
|---|---|---|---|---|---|---|---|---|---|---|
| | | GPT (Units) | GOT (Units) | LDH (units) | ALP^{b} | TG (mg %) | Albumin (g %) | LP^{c} | GSH^{d} | Protein (mg %) |
| Vehicle | - | 125.82±4.32 | 134.23±3.94 | 186.71±9.33 | 27.72±2.16 | 31.64±2.28 | 4.00±0.09 | 31.49±2.34 | 6.22±0.18 | 178.55±9.63 |
| Veh+APAP | - | 1510.82±50.02 | 1138.76±83.96 | 1887.30±45.5$ | 62.22±3.38 | 74.21±1.96 | 2.66±0.23 | 70.95±3.37 | 2.14±0.13 | 129.92±4.89 |
| AP only (Per-se) | 50 | 126.11±3.09 | 134.92±4.25 | 181.02±10.29 | 27.72±2.71 | 32.29±2.31 | 3.96±0.08 | 31.60±2.09 | 6.40±0.21 | 182.05±10.23 |
| AP+ APAP | 6.25. | 1224.99±63.65 | 992.69±66.36 | 1454.48±66.02 | 55.84±2.27 | 63.92±2.11 | 3.10±0.08 | 58.96±2.04 | 2.85±0.19 | 133.20±5.39 |
| | | (20.76) | (14.54) | (25.45) | (18.49) | (24.17) | (32.84) | (10.61) | (17.40) | (6.75) |
| AP+APAP | 12.5 | 1051.99±46.30 | 816.54±47.10 | 1255.65±61.32 | 49.41±1.35 | 56.59±2.42 | 3.29±0.15 | 53.10±2.07 | 3.78±0.19 | 141.36±4.25 |
| | | (33.12) | (32.07) | (37.14) | (37.13) | (41.39) | (47.02) | (46.23) | (40.19) | (23.52) |
| AP+ APAP | 25 | 881.23±36.85 | 720.76±50.62 | 1014.27±44.04 | 43.92±2.19 | 51.68±2.73 | 3.32±0.15 | 50.62±2.23 | 4.15±0.15 | 148.36±3.57 |
| | | (45.45) | (41.40) | (51.34) | (53.04) | (52.92) | (49.25) | (51.45) | (49.26) | (37.92) |
| AP+ APAP | 50 | 760.73±49.35 | 534.56±19.65 | 952.45±34.82 | 40.18±2.03 | 49.22±1.78 | 3.55±0.18 | 47.90±1.93 | 4.400±0.25 | 155.34±3.11 |
| | | (54.15) | (60.14) | (54.97) | (63.88) | (58.70) | (66.41) | (58.35) | (57.35) | (52.27) |
| AP+ APAP | 100 | 764.37±44.32 | 795.92±27.59 | 1256.05±33.97 | 44.14±2.24 | 47.39±2.13 | 3.38±0.26 | 45.30±2.08 | 4.60±0.24 | 146.78±5.09 |
| | | (53.89) | (34.12) | (37.11) | (52.40) | (63.00) | (53.73) | (64.95) | (60.29) | (34.66) |
| Sily+ APAP | 50 | 797.16±62.96 | 605.95±45.90 | 917.04±31.08 | 43.76±2.08 | 51.00±2.61 | 3.43±0.20 | | 4.53±0.21 | 156.15±3.44 |
| | | (51.52) | (53.04) | (57.05) | (53.51) | (54.52) | (57.46) | 48.46±1.95 | (58.57) | (53.94) |
| | | | | | | | | (56.92) | | |

| | | | | | | | | | | |
|---|---|---|---|---|---|---|---|---|---|---|
| a : Values represent mean ± SE of six animals in each group; Units : Each unit is µ mole pyruvate/min /L; b : µ mole of p-nitrophenol formed /min/L c: lipid peroxidation (n mole MDA/g liver); d: glutathione (µ mole GSH/g liver). | | | | | | | | | | |

**Table-3: Hepatoprotective potential of HV-392 against Paracetamol (APAP) induced hepatic injury in Rodents (Prophylactic).**

| Treatment | Dose mg/kg | Serum parameter^{a} | | | | | | Hepatic parameters | | |
|---|---|---|---|---|---|---|---|---|---|---|
| | | GPT (Units) | GOT (Units) | LDH (units) | ALP^{b} | TG (mg %) | Albumin (g %) | LP^{c} | GSH^{d} | Protein (mg %) |
| Vehicle | - | 183.05±1 4.81 | 167.40±8.94 | 196.44±6.51 | 25.38±2.02 | 29.83±1.82 | 3.32±0.20 | 30.52±2.05 | 7.04±0.37 | 175.25±6.51 |
| Vehicle + APAP | - | 1758.95± 59.21 | 1078.90±43.07 | 1736.78±54.81 | 57.25±2.54 | 60.99±2.14 | 2.02±0.09 | 62.60±2.21 | 2.99±0.09 | 133.86±2.40 |
| HV-392 only (Per se) | 50 | 182.30±1 0.97 | 169.97±9.23 | 190.76±9.07 | 26.94±2.69 | 29.84±1.44 | 3.26±0.23 | 27.74±2.58 | 7.15±0.27 | 176.79±5.28 |
| HV-392 | 6.25. | 1026.59± | 708.07±30.63 | 1278.09±65.20 | 44.62±2.00 | 58.02±2.96 | 2.31±0.21 | 51.02±1.41 | 3.44±0.21 | 141.58±3.82 |
| + APAP | | 49.71 | (40.47) | (29.77) | (39.62) | (9.53) | (22.30) | (46.09) | (11.11) | (13.65) |
| | | (46.74) | | | | | | | | |
| HV-392 + | 12.5 | 836.30±5 | 571.68±52.41 | 973.42±45.23 | 38.26±1.33 | 47.02±1.71 | 2.45±0.19 | 46.50±2.01 | 3.97±0.24 | 146.39±3.74 |
| APAP | | 3.17 | (55.49) | (49.55) | (59.58) | (44.83) | (33.00) | (50.18) | (24.19) | (30.27) |
| | | (58.51) | | | | | | | | |
| HV-392 + | 25 | 655.73±7 | 497.53±46.41 | 746.87±64.58 | 36.63±1.97 | 43.86±2.36 | 2.80±0.09 | 42.15±1.61 | 5.16±0.27 | 152.11±3.00 |
| APAP | | 5.82 | (63.65) | (64.26) | (64.70) | (54.97) | (60.00) | (63.75) | (53.58) | (44.09) |
| | | (70.00) | | | | | | | | |
| HV-392 | 50 | 483.63±2 | 430.82±54.76 | 488.91±41.15 | 30.23±2.90 | 42.37±2.64 | 3.17±0.21 | 37.90±1.26 | 5.93±0.30 | 157.05±2.41 |
| + APAP | | 9.09 | (70.99) | (81.01) | (84.78) | (59.75) | (88.46) | (76.99) | (73.82) | (56.02) |
| | | (80.92) | | | | | | | | |
| Silymarin | 50 | 819.01±5 | 561.26±38.39 | 848.05±46.66 | 40.84±2.30 | 44.02±2.10 | 2.65±0.15 | 44.50±1.97 | 5.21±0.32 | 153.23±3.51 |
| + APAP | | 7.95 | (56.64) | (57.69) | (51.49) | (54.46) | (48.46) | (56.42) | (54.82) | (46.79) |
| | | (59.64) | | | | | | | | |

| | | | | | | | | | | |
|---|---|---|---|---|---|---|---|---|---|---|
| a: Values represent mean ± SE of six animals in each group; Units : Each unit is µ mole pyruvate/min /L; b : µ mole of p-nitrophenol formed /min/L c: lipid peroxidation (n mole MDA/g liver); d: glutathione (µ mole GSH/g liver). | | | | | | | | | | |

**Table-4: Hepatoprotective activity (in vivo) of TCA and silymarin (Pre-treatment) fed at 72h, 48h, 24h, 1h before inhalation of diethyl - ether and 1h after Acetaminophen (APAP) 200mg/kg given i.p. 6h after exposure to diethyl - ether (prophylactic study).**

| **Treatment** | Dose | Serum parameters | | | | | Hepatic parameters | |
|---|---|---|---|---|---|---|---|---|
| | mg/kg p.o. | GPT (Units) | GOT (Units) | ALP^{b} | Bilirubin (mg %) | Triglycerides (mg %) | Lipid peroxidation^{c} | Glutathione^{d} |
| TCA+ APAP | 50 | 57.99 | 54.92 | 52.96 | 56.35 | 66.05 | 55.48 | 42.35 |
| Silymarin + APAP | 50 | 52.12 | 44.19 | 46.63 | 53.11 | 41.91 | 61.44 | 55.10 |

| | | | | | | | | |
|---|---|---|---|---|---|---|---|---|
| a5Values represent the mean ± SE and within parentheses percent hepatoprotective activity of six animals in each group, Mice: Swiss albino (25-30 g). Unit: each unit is µmole pyruvate/min/L. b: is µ mole of *p*-nitrophenol formed/min/ L, c: is n moles MDA/g liver., d: is µ mole GSH/g liver | | | | | | | | |

**Table -5 Prophylactic effect of hepatoprotective formulation HV-392 against paracetamol induced toxicity**

| **Drug+dose** | **Centrilobular Necrosis** | **Bridging Necrosis** | **Spotty Necrosis** | **Ballooning degeneration of hepatocytes** | **Lobular Disarray** | **Chronic LMN, Cell infiltrates** |
|---|---|---|---|---|---|---|
| Toxin i.e., | + + + | + + | + + | + + | + + | + |
| APAP 200 mg only | | | | | | |
| HV-392 | ++ | - | + | ++ | ++ | + |
| 6.25 mg/kg | | | | | | |
| +AFAP 50 mg/kg | | | | | | |
| HV-392 | +++ | - | + | ++ | ++ | + |
| 12.5 mg/kg | | | | | | |
| +APAP 50 mg/kg | | | | | | |
| HV-392 | - | - | + | + | - | + |
| 250 mg/kg | | | | | | |
| +APAP 50 mg/kg | | | | | | |
| HV-392 | - | - | + | - | - | + |
| 50 mg/kg | | | | | | |
| +APAP 50 mg/kg | | | | | | |
| Silymarin 50 mg/kg - | | - | - | - | - | + |
| +APAP 50 mg/kg | | | | | | |

| | | | | | | |
|---|---|---|---|---|---|---|
| +++ Marked ++ Moderate + Mild - Absent | | | | | | |

## Claims

1. A synergistic formulation comprising trans-tetracos-15-enoic acid (t-TCA) and andrographolide (AP) in the ratio ranging between 1:7 to 7:1 (w/w), useful in hepatoprotection

2. A synergistic formulation as claimed in claim 1, wherein the ratio of t-TCA and AP is 1:1 (w/w).

3. A synergistic formulation as claimed in claim 1, wherein the constituents of the formulation can be either herbal or synthetic.

4. A synergistic formulation as claimed in claim 1, wherein the formulation shows therapeutic index of more than 40.

5. A process for the preparation of a synergistic formulation comprising trans-tetracos-15-enoic acid (t-TCA) and andrographolide (AP) useful in hepatoprotection, said process comprising steps of:
a, grounding the particles of the t-TCA and AP into fine particles,
b. mixing the fine particles in ratio ranging between 1:7 to 7:1 (w/w), and
c. grinding the mixture to obtain formulation.

6. A process as claimed in claim 5, wherein grounding the particles for about 20 minutes at about 100-120 rotation/minute.

7. A process as claimed in claim 5, wherein grinding the mixture for about 15 minutes at about 70-80 rotations/minute.

8. A process as claimed in claim 5, wherein the formulation shows the mesh size of about less than 100-mesh.

9. A process as claimed in claim 5, wherein the constituents of the formulation can be either herbal or synthetic.

## Patentansprüche

1. Synergistische Formulierung, umfassend Trans-Tetracos-15-ensäure (t-TCA) und Andrographolid (AP) im Verhältnis zwischen 1:7 und 7:1 (w/w), die beim Leberschutz nützlich ist.

2. Synergistische Formulierung wie in Anspruch 1 beansprucht, wobei das Verhältnis von t-TCA bis AP 1:1 (w/w) beträgt.

3. Synergistische Formulierung wie in Anspruch 1 beansprucht, wobei die Bestandteile der Formulierung entweder pflanzlich oder synthetisch sein können.

4. Synergistische Formulierung wie in Anspruch 1 beansprucht, wobei die Formulierung eine therapeutische Breite von mehr als 40 aufweist.

5. Verfahren zur Herstellung einer synergistischen Formulierung, umfassend Trans-Tetracos-15-ensäure (t-TCA) und Andrographolid (AP), die beim Leberschutz nützlich ist, wobei das Verfahren die folgenden Schritte umfasst:
a. das Zermahlen von t-TCA und AP in feine Partikel,
b. das Vermischen der feinen Partikel in einem Verhältnis zwischen 1:7 und 7:1 (w/w), und
c. das Mahlen der Mischung, um die Formulierung zu erhalten.

6. Verfahren wie in Anspruch 5 beansprucht, wobei das Zermahlen der Partikel für etwa 20 Minuten mit etwa 100-120 Umdrehungen/Minute erfolgt.

7. Verfahren wie in Anspruch 5 beansprucht, wobei das Mahlen der Mischung für etwa 15 Minuten mit etwa 70-80 Umdrehungen/Minute erfolgt.

8. Verfahren wie in Anspruch 5 beansprucht, wobei die Formulierung etwa eine Maschenweite von weniger als 100 Mesh aufweist.

9. Verfahren wie in Anspruch 5 beansprucht, wobei die Bestandteile der Formulierung entweder pflanzlich oder synthetisch sein können.

## Revendications

1. Formulation synergique comprenant de l'acide trans-tétracos-15-énoïque (t-TCA) et de l'andrographolide (AP) en un rapport situé dans la plage allant de 1/7 à 7/1 (pds/pds), utile dans la protection du foie.

2. Formulation synergique selon la revendication 1, dans laquelle le rapport du t-TCA sur l'Ap est de 1/1 (pds/pds).

3. Formulation synergique selon la revendication 1, dans laquelle les constituants de la formulation peuvent provenir de plantes médicinales ou être synthétiques.

4. Formulation synergique selon la revendication 1, dans laquelle la formulation présente un indice thérapeutique supérieur à 40.

5. Processus de préparation d'une formulation synergique comprenant de l'acide trans-tétracos-15-énoïque (t-TCA) et de l'andrographolide (AP) utile dans la protection du foie, ledit processus comprenant les étapes consistant à :
a. broyer les particules du t-TCA et de l'AP en particules fines,
b. mélanger les particules fines au rapport situé dans la plage allant de 1/7 à 7/1 (pds/pds), et
c. broyer le mélange pour obtenir la formulation.

6. Processus selon la revendication 5, dans lequel les particules sont broyées pendant environ 20 minutes à environ 100 à 120 tours/minute.

7. Processus selon la revendication 5, dans lequel le mélange est broyé pendant environ 15 minutes à environ 70 à 80 tours/minute.

8. Processus selon la revendication 5, dans lequel la formulation présente une taille de mesh inférieure à environ 100 meshes.

9. Processus selon la revendication 5, dans lequel les constituants de la formulation peuvent provenir de plantes médicinales ou être synthétiques.
